# EUROPEAN PATENT APPLICATION

(11) **EP 3 705 026 A1**
(43) Date of publication of application: **09.09.2020**
(21) Application number: 18870582.6
(22) Date of filing: 16.10.2018
(51) Int. Cl.: A61B 1/267, A61B 1/005, A61B 1/05

(54) **MECHANICAL LARYNGOSCOPE WITH AN AUTOMATIC OR MANUAL LEVER MECHANISM TO FACILITATE TRACHEAL INTUBATION**

(30) Priority: 23.10.2017 BR 102017022831
(71) Applicant: Clemente Pereira, Gilberto, 74883-015 Goiânia (BR)
(72) Inventor: Clemente Pereira, Gilberto, 74883-015 Goiânia (BR)
(74) Representative: Sach, Greg Robert
(86) International application number: PCT/BR2018/050379
(87) International publication number: WO 2019/079872

(57) **Abstract**

The present invention relates to a manual (1) and automatic (2) laryngoscope provided with a lower transmission shaft (10), an upper transmission shaft (9) and a lever (8) for resting on the palate (3), the manual laryngoscope (1) further having an actuating lever (4) with a knuckle joint (4a) and torsion spring (Mt) in addition to a keeper (7) on the handle (12), this handle (12) having an internal compartment for housing alkaline, C2 batteries, and the automatic laryngoscope (2) also having a servomotor (20) with three electrical terminals, a compartment (19), upper transmission (17) and lower transmission (16), which are connected to the auxiliary paddle (15) and to the paddle of the blade (14) on the auxiliary shaft (13), moving transmission, upper and lower (9 and 10) shafts and the lever (8) resting on the palate (3).

## Description

This invention patent refers to a laryngoscope equipped with a system of levers which, when actuated, facilitate the opening of the oral cavity and the view of the glottis, and consequently tracheal intubation. The levers totally or partially decrease the force exerted by the user, being actuated manually or by means of an electro-electronic actuation mechanism.

Some models of laryngoscope are known in the state of the art. Several models of video-laryngoscopes are also known. For the invention in question, to facilitate the view of the epiglottis, a route has been developed through the mechanical laryngoscope with automatic actuation that allows optical cables to be used with a connected video system. Models which already exist on the market were selected. In order for the system to preserve its sterilisable capacity and support the largest possible number of video systems, the endoscopic camera is covered by a surgical tube, made of translucent material, with an internal diameter of at least 5.5 mm and a maximum external diameter of 10 mm. This said, the solution will include commercial systems where the endoscopic camera has a diameter between 3.3 mm and 6 mm. Examples of compatible systems include the Clarus Pocket Scope, Teslong NTS 150 RS and Giraffecam 1.0 Soft ShortFocus. However, no laryngoscopes are known that are equipped with actuation mechanisms using an actuating lever and other levers, to rest on the palate and move the epiglottis out of the way.

The state of the art is also known to include US patent 2017/0181614, which concerns a laryngoscope with a palate rest, connected to the blade by a number of arms, pushed against the roof of the mouth and opening the airway to facilitate the view of the vocal cords.

It is worth noting that, as it contains a number of arms (5a, 5b and 5c) all arranged externally to the blade, it presents difficulties both in manufacture and in use inside the patient's mouth. However, the product's commercialisation is unknown. The laryngoscope in this invention patent is differentiated by having two levers, acting inside the mouth in opposite directions, imitating the opening movement of a clamp. In addition, the entire transmission mechanism is located inside the blade, and can be actuated either manually or automatically by electronic means.

On the other hand, McCoy's laryngoscope, based on the standard Macintosh blade, is widely known in the market. Its main feature is the articulated tip, which is manipulated by a lever on the back of the instrument. The McCoy thereby facilitates the removal of the epiglottis to view the glottis.

However, the McCoy laryngoscope moves only the tip of the blade, whereas the laryngoscope in question here has a longer, articulated part which, when actuated, removes the epiglottis and other structures, e.g. the base of the tongue and anterior wall of the pharynx. This therefore results in greater utility and ease of handling.

It is also worth mentioning that the instruments listed above do very little to reduce the user's effort, still requiring a great effort for intubation, and leading to user fatigue in the case of repeated attempts. However, with this invention the effort will be reduced significantly, using the manual mechanism, or even totally, using the automatic mechanism. It is important to note that the forces required have been transferred into the mouth.

One of the existing problems this invention patient intends to solve relates to the fact that when a tracheal intubation is performed, one of the greatest concerns is always linked to the success of the procedure and its proper maintenance, with the presence of a difficult airway being one of the major challenges. Inadequate handling of the airway is the most frequent cause of complications related to the speciality (anaesthesiology) and is responsible for 30% of deaths from an exclusively anaesthetic cause.

The professional defence committee of the American Society of Anesthesiologists (ASA) conducted a review which analysed lawsuits filed against anaesthesiologists and resolved by agreement. This research revealed that the vast majority of adverse events were related to the lungs and airways.

Three causes - difficult ventilation (38%), failure to recognise oesophageal intubation (17%), and the difficulty or even impossibility of intubation (18%) - were responsible for 75% of complications. Death or brain damage occurred in 85% of these cases, most of which were caused by error or omission, e.g. failure to immediately recognise the severity of the problem, lack of careful observation of the airways, and not acting correctly and promptly.

The main consequences associated with improperly handling the difficult airway are: death, brain injury, cardiopulmonary arrest, unnecessary tracheostomy, trauma to the airway or the teeth.

The difficult airway is defined as a clinical situation where a trained doctor has difficulty intubating the patient, maintaining manual ventilation, or both.

Difficult laryngoscopy is the non-visibility of any part of the vocal cords with the use of conventional laryngoscopy.

The purpose of laryngoscopy is to expose the glottis in such a way that tracheal intubation is possible. The expected view is of a cylindrical structure, with a central slit in the shape of an inverted "V", whose edges are the vocal cords through which the larynx is viewed. Sometimes, due to anatomical difficulties, this image is restricted according to which laryngeal structures can or cannot be observed during a laryngoscopy. Cormack and Lehane proposed a practical classification in 1984.

In their classification, laryngoscopy is divided into four categories according to the structures visible:
► Class I - Glottis clearly visible;
► Class II - Only the posterior part of the glottis can be seen;
► Class III - Only the epiglottis can be seen, no portion of the glottis is visible;
► Class IV - Neither the epiglottis nor the glottis can be seen.

Several factors are important for laryngoscopy to be successful, such as: jaw mobility; neck mobility, diameter and length; mouth opening; length of the upper incisor teeth; conformation of the palate; tongue size; etc.

The present invention was therefore developed with the purpose of solving such problems and difficulties. It will be better detailed with reference to the attached drawings, where:
**Drawing 1** shows a side view of the manually actuated laryngoscope with a lateral opening, so it is possible to see the actuation mechanism, the palate-resting lever and the distal blade, which act as levers.
**Drawing 2** shows the same view as Drawing 1, but with the manually actuated laryngoscope turned on the opposite side.
**Drawing 3** shows a perspective view of the manually actuated laryngoscope shown in Drawing 1.
**Drawing 3a** shows a perspective view of the blade attached to the laryngoscope's upper cover, with two different sizes and curvatures.
**Drawing 4** shows the same view as Drawing 1, but with the laryngoscope's lever system actuated and the actuating lever locked to the handle.
**Drawing 5** shows an anterior oblique perspective view of the laryngoscope automatically actuated by an electro-electronic mechanism.
**Drawing 6** shows a side view of the automatically actuated laryngoscope.
**Drawing 7** shows an oblique rear view of the manually actuated laryngoscope.
**Drawing 7a** shows an oblique rear view of the automatically actuated laryngoscope.
**Drawing 8** shows a side view of the set of levers and handles of the manually actuated laryngoscope.
**Drawing 8a** shows a section of the knuckle joint of the actuation mechanism, to display the torsional spring of this lever.
**Drawing 9** illustrates the direction, during operation, of the levers and transmission shafts of the manually actuated laryngoscope.
**Drawing 10** illustrates the direction, during operation, of the levers and transmission shafts of the automatically actuated laryngoscope.
**Drawing 11** shows a side view of the blades, opposite the actuation mechanism, and only a part of the automatic actuation mechanism.
**Drawing 12** shows a perspective view of the servomotor and its compartment, together with the servo paddle (18) that performs the rotational movements that move the levers.
**Drawing 13** illustrates the movement during operation of the automatic actuation mechanism.
**Drawing 14** shows the assembly of the automatic actuation mechanism with the palate rest and the servomotor.
**Drawing 15** shows the entire automatic actuation mechanism, together with the palate rest.
**Drawing 15a** shows an "exploded" image of the central part of the actuation mechanism.

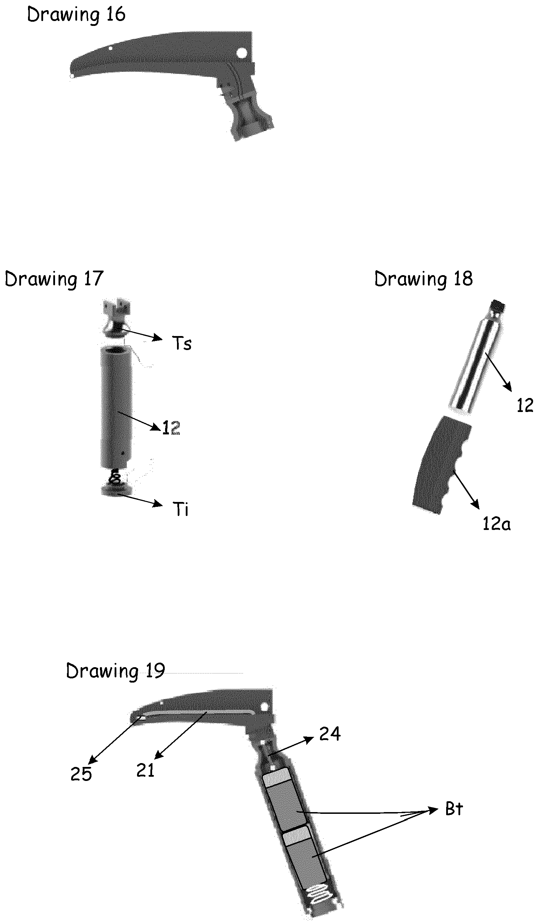

**Drawing 16** shows the proximal blade in section, with the view of the channel the wires pass through for electrical conduction of the lighting system.
**Drawing 17** shows the laryngoscope handle detached from the lower (Ti) and upper (Ts) covers, which the internal battery(ies) fit into.
**Drawing 18** shows the manual laryngoscope handle detached from the silicone grip that covers it.
**Drawing 19** shows the laryngoscope with lateral section, displaying the electrical part of the handle and the proximal blade responsible for taking light to the distal part of the proximal blade.

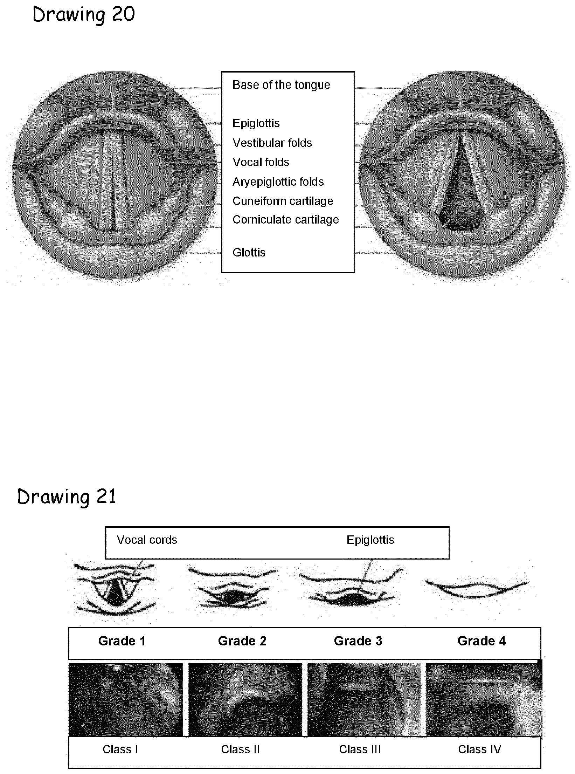

**Drawing 20** shows the expected view when the purpose of laryngoscopy is to expose the glottis so tracheal intubation is possible. There is a cylindrical structure with a central slit in the shape of an inverted "V", whose edges are the vocal cords through which the larynx is seen.
**Drawing 21** shows the classification proposed by Cormack and Lehane with the laryngeal structures that can generally be observed during a laryngoscopy.

As can be seen in the attached drawings, this invention involves a laryngoscope with manual or automatic actuating levers, with the automatic one having an electro-electronic actuation system. Both the manual and the automatic ones will reduce the difficulty of tracheal intubation, especially for patients considered "difficult to intubate", reducing the damage caused by intubation difficulty, while also requiring less effort from the user.

As seen in the attached drawings, the manually actuated laryngoscope (1) has a proximal blade (5) and distal blade (6), lower transmission shaft (10), upper transmission shaft (9) and palate-resting lever (8) located diagonally from the proximal blade (5), with this lever being connected to the palate rest (3) via a joint.

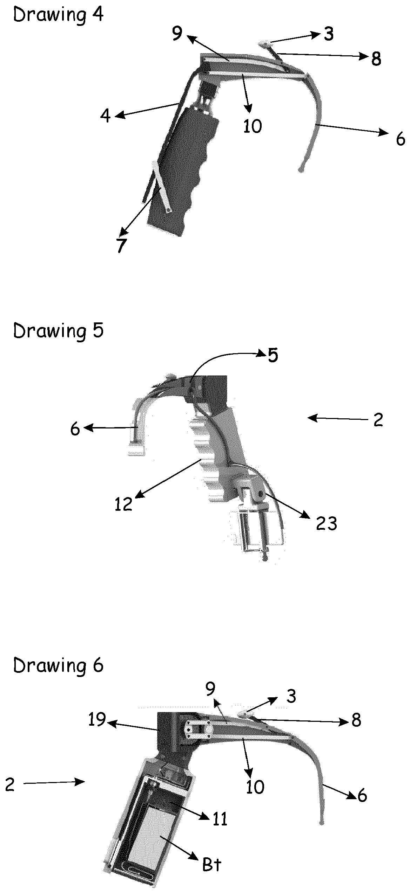

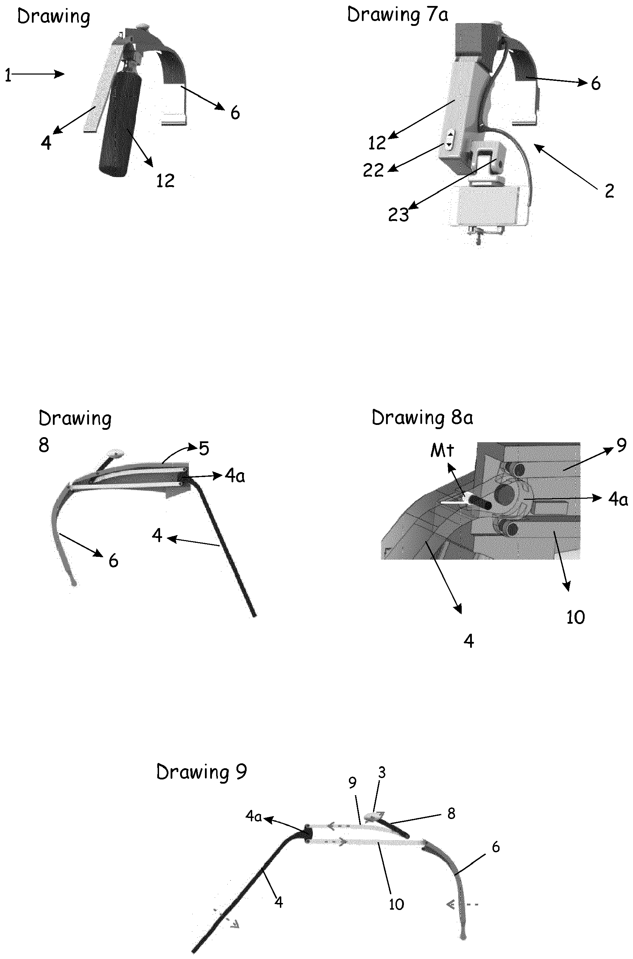

The manual laryngoscope (1) also has an actuating lever (4) and a keeper (7) which can be put in several different positions, adapting to different patients and situations. The handle (12) has an internal compartment to house two C2 alkaline batteries, to supply the lighting system composed of an LED lamp (25), with electrical conduction via a system of internal and external wires (21 and 24), from the handle to the end of the proximal blade. It also has a silicone grip (12a) used to cover the handle (12), thereby improving the ergonomics. The operating mechanism of the manual laryngoscope (1) involves pressing the lever (4) towards the handle
(12) which, being connected to the base of the proximal blade (5) and having a knuckle joint (4a) attached to the upper and lower transmission shafts (9 and 10), provides the back-and-forth movement of these shafts (9 and 10). This pushes up the palate-resting lever (8), placing the palate rest (3) against the roof of the mouth (hard palate), and produces the movement in the opposite direction of the other intra-oral lever formed by the distal blade (6), as seen in Drawings 4, 8 and 9. The knuckle joint (4a) is fixed with a torsional spring (Mt) as seen in Drawing 8a, with the function of returning the lever to the rest position when it is not being pressed.

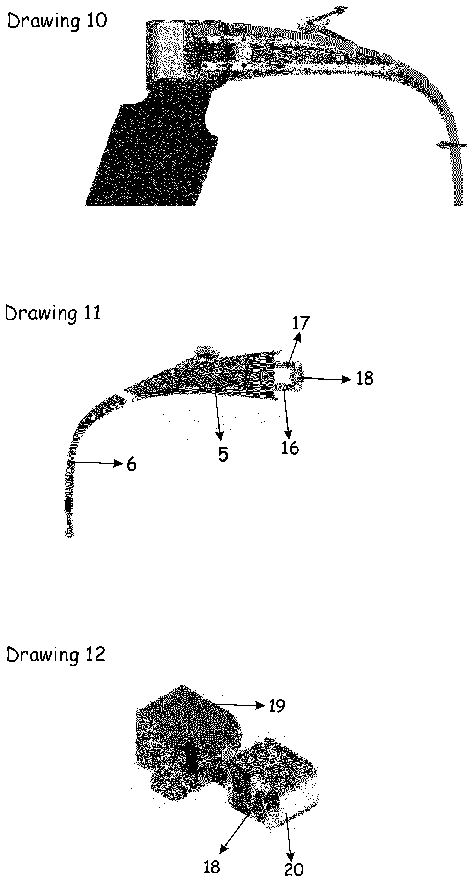

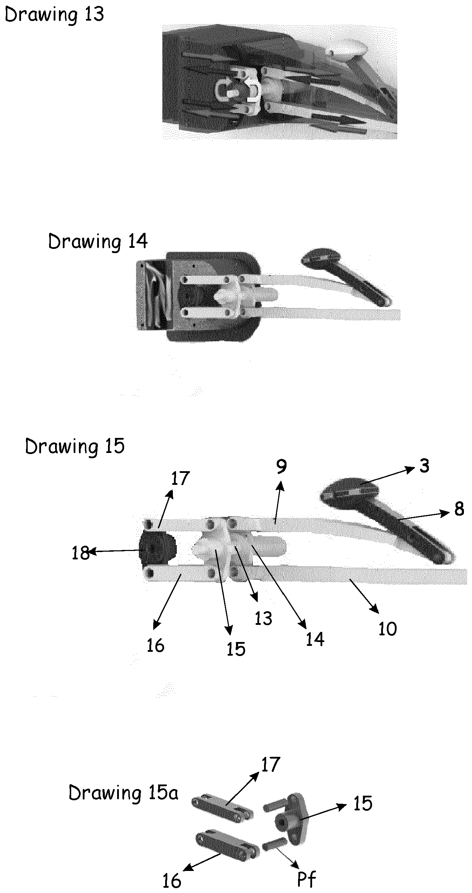

The automatic laryngoscope (2) consists of a servomotor (20) housed in a compartment (19) arranged to interconnect the handle (12) and the proximal blade (5). This servomotor (20) is actuated by actuation buttons (22) which, through the servo paddle (18), actuate the upper transmission (17) and lower transmission (16). These, being connected to an auxiliary paddle (15) and the blade paddle (14) by an auxiliary shaft (13), provide the back-and-forth movement to the upper and lower transmission shafts (9 and 10). In the same step that the upper transmission shaft (9) is pulled, it also pulls the palate-resting lever (8), increasing its angle, thereby raising the palate rest (3).
It then goes in the opposite direction, pushing the lower transmission shaft (10) in order to push the upper part of the distal blade (6). This makes it open the patient's glottis and push the base of the tongue, as shown in the movements indicated in Drawings 10, 13, 14 and 15.

The servomotor (20) has three electrical terminals: two for its supply and the third for receiving its control signal. To power the device, a system has been designed that can be used with a rechargeable battery (Bt) or DC power supply connected to the mains. To control the servomotor, a printed circuit board has been designed, based on a microcontroller.

The lighting system consists of a rechargeable battery (Bt) inside a cartridge (11) housed in the handle (12). It has the function of powering the LED lamp (25) located on the proximal blade (5). The system is actuated by a button located on the handle and conducted to the lamp by a conduction system (21 and 25) similar to that of the manual laryngoscope (1).

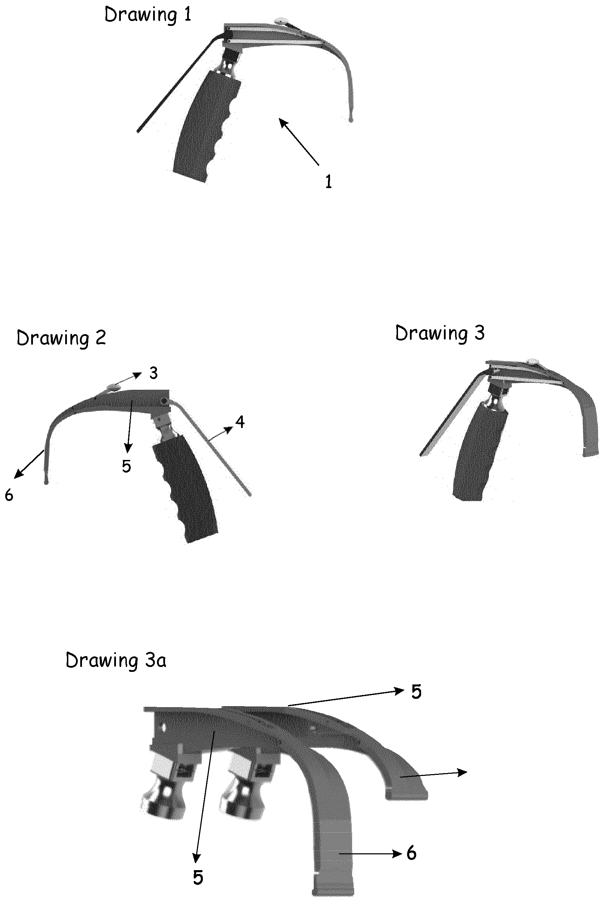

The laryngoscope with levers to facilitate tracheal intubation has an automatic version (Drawing 5) and a manual version (Drawing 1). The automatic version contains a camera support (23) for using a video system if desired.

Of course, the models presented in the attached drawings are not exhaustive: this laryngoscope, automatic or manual, can be produced in different designs, with different shapes and sizes of blades, thereby preserving the differential i.e. the levers.

## Claims

1. Mechanical laryngoscope with an automatic or manual lever mechanism to facilitate tracheal intubation, to be used in tracheal intubation to facilitate the view of the glottis, is characterised as comprising a laryngoscope (1 and 2) containing a movement system for the distal blade (6) and lever (8) for the palate rest (3) arranged internally to the proximal blade (5).

2. Mechanical laryngoscope with an automatic mechanism, according to claim 1, is **characterised by** the movement of the distal blade (6) and the lever (8) with the palate rest (3). This is composed of a servomotor (20) housed in the compartment (19) connecting the handle (12) and the proximal blade (5) in order to actuate the servo paddle (18), the upper transmission (17) and the lower transmission (16). These are both connected to the auxiliary paddle (15) and the blade paddle (14) by an auxiliary shaft (13), in order to provide back-and-forth movements to the upper transmission shafts
(9) and the lower transmission shaft (10), pulling the lever (8) and the palate rest (3) back and forth, as well as the distal blade (6).

3. Mechanical laryngoscope with an automatic mechanism, according to Claims 1 and 2, is **characterised by** the lighting system and electro-electronic actuation by means of a battery (Bt) housed in a cartridge (11) inside the handle (12). This battery supplies both the lighting system with LED lamp (25) via electrical conduction through internal and external wires (21 and 24), and the servomotor by means of a PCI card.

4. Mechanical laryngoscope with a manual mechanism, according to Claim 1, is **characterised by** the movement system inside the proximal blade (5) consisting of a lower
transmission shaft (10), upper transmission shaft (9) and lever (8) with palate rest (3).

5. Mechanical laryngoscope with a manual mechanism, according to Claims 1 and 4, is **characterised by** manual actuation through an actuating lever (4) which has, at the top, a knuckle joint (4a) fixed to the upper and lower transmission shafts (9 and
10) and a torsional spring (Mt) and, at the bottom, a set of teeth (dt) for fixing the keeper (7).

6. Mechanical laryngoscope with a manual mechanism, according to Claims 1, 4 and 5, is **characterised by** the manual laryngoscope (1) with a handle (12) containing an internal compartment for batteries (Bt), lighting system with LED lamp (25) and electrical conduction through internal and external wires (21 and 24), in addition to a silicone grip (12a).
